# EUROPÄISCHE PATENTSCHRIFT

(11) **EP 0 847 726 B1**
(45) Veröffentlichungstag und Bekanntmachung des Hinweises auf die Patenterteilung: **05.03.2003**
(21) Anmeldenummer: 98100509.3
(22) Anmeldetag: 27.08.1994
(51) Int. Cl.: A61B 5/15, B01L 3/14

(54) **Blutentnahmevorrichtung**
Blood collecting device
Dispositif de prise de sang

(30) Priorität: 22.09.1993 DE 4332189
(43) Veröffentlichungstag der Anmeldung: 17.06.1998
(62) Teilanmeldung aus: 94113478.5
(73) Patentinhaber: KABE Labortechnik GmbH, 51588 Nümbrecht-Elsenroth (DE)
(72) Erfinder: Hardt, Stefan, Dipl.-Ing., 34225 Baunatal 6 (DE); Finis, Frank, Dipl.-Ing., 34466 Wolfhagen (DE)
(74) Vertreter: Selting, Günther, Dipl.-Ing.

(56) Entgegenhaltungen:
- EP-A- 0 031 900
- DE-A- 2 328 718
- DE-A- 2 603 963
- US-A- 2 722 257
- US-A- 4 192 320

## Beschreibung

Die Erfindung betrifft eine Blutentnahmevorrichtung mit einem Röhrchen, in dem ein Trennkörper angeordnet ist, dessen spezifisches Gewicht zwischen den spezifischen Gewichten der zu trennenden Blutkomponenten liegt.

Derartige Blutentnahmevorrichtungen sind bekannt aus DE 27 11 336 C2, DE 27 34 720 oder EP 0 056 609. Sofern diese Blutentnahmevorrichtungen ein Röhrchen aus relativ weichem Kunststoff verwenden, welches beim Zentrifugieren unter der Kraft der in ihm enthaltenen Flüssigkeit aufgeweitet wird und um den Trennkörper herum einen Spalt freigibt, ist die Weichheit des Röhrchens oftmals nachteilig. Um einerseits ein weiches Röhrchen verwenden zu können und andererseits die Blutentnahmevorrichtung gegen äußere Einwirkungen widerstandsfähig zu machen, ist in EP 0 305 459 B1, von der der Oberbegriff des Patentanspruchs 1 ausgeht, eine Blutentnahmevorrichtung beschrieben, die ein relativ steifes Außenröhrchen und ein darin enthaltenes, relativ weiches Innenröhrchen aufweist, das sich beim Zentrifugieren aufweiten kann. Zwischen Außenröhrchen und Innenröhrchen besteht ein Ringspalt und das Innenröhrchen enthält einen Trennkörper, der mit einer abbrechbaren Kolbenstange versehen ist und somit gleichzeitig als Kolben zum Einziehen einer Blutprobe in die Blutentnahmevorrichtung verwendet werden kann. In dem Kanülenansatz des Außenröhrchens kann ein das Rückströmen des Blutes verhinderndes Ventil vorgesehen sein oder ein von der Kanüle zu durchstechender Stopfen. Wenn der Kanülenansatz eine von der Kanüle zu durchstechende Membran enthält, besteht beim Zentrifugieren die Gefahr, dass durch die Kraft der im Innenröhrchen enthaltenen Flüssigkeitssäule Flüssigkeit an der Membran entlang austritt.

Eine Blutentnahmevorrichtung, von der der Oberbegriff des Patentanspruchs 1 ausgeht, ist bekannt aus US-A-2,722,257. Diese Blutentnahmevorrichtung weist ein Röhrchen mit einem Kanülenansatz auf, auf dem eine Kanüle abnehmbar befestigt wird. Auf den Kanülenansatz kann ein Verschlussstück aufgesetzt werden, das einen in den Kanülenansatz eintauchenden Zapfen sowie eine den Kanülenansatz umschließende Umfangswand aufweist. Das Verschlussstück dient einerseits zum Verschließen des Röhrchens und andererseits zum Zentrieren des Röhrchens in einem Zentrifugenbecher.

EP 0 031 900 A1 beschreibt eine Blutentnahmevorrichtung mit einem Röhrchen, das mit einer durchstechbaren Membran verschlossen ist. Auf der Außenseite der Membran ist eine starre Scheibe mit einer Mittelbohrung aufgelegt, die mit dem Röhrchen fest verbunden ist.

Der Erfindung liegt die Aufgabe zugrunde, eine Blutentnahmevorrichtung zu schaffen, bei der die Gefahr des unbeabsichtigten Flüssigkeitsaustritts in der Zentrifuge beseitigt ist.

Die Lösung dieser Aufgabe erfolgt erfindungsgemäß mit den Merkmalen des Patentanspruchs 1.

Nach der Erfindung ist ein Zentrifugeneinsatz vorgesehen, der eine Aufnahme für den eine durchstechbare Membran enthaltenden Kanülenansatz aufweist, wobei in der Aufnahme ein Zapfen zum Abstützen der Membran angeordnet ist. Dadurch wird der Kanülenansatz mit der Membran an der Einstichstelle während der Zentrifugation verstärkt abgedichtet. Gleichzeitig stützt der Zentrifugeneinsatz das Außenröhrchen ab und verhindert, dass der Kanülenansatz infolge der Zentrifugalkraft axial zusammengedrückt wird. Der Zentrifugeneinsatz kann als separates Teil in den Zentrifugenbecher eingesetzt werden, er kann jedoch auch fest im Zentrifugenbecher enthalten sein.

Im folgenden wird unter Bezugnahme auf die Zeichnungen ein Ausführungsbeispiel der Erfindung näher erläutert.

Es zeigen:
- Fig. 1: einen Längsschnitt der Blutentnahmevorrichtung;
- Fig. 2: in vergrößertem Maßstab einen Teil-Längsschnitt durch das vordere Ende des Außenröhrchens;
- Fig. 3: einen Teil-Längsschnitt durch das vordere Ende des Innenröhrchens;
- Fig. 4: einen Schnitt durch das vordere Ende der zusammengesetzten Röhrchen;
- Fig. 5: eine Variante, bei der die Erhebung am Innenröhrchen vorgesehen ist;
- Fig. 6: einen Teil-Längsschnitt des rückwärtigen Endes des Innenröhrchens;
- Fig. 7: eine Ansicht des Trennkörpers;
- Fig. 8: eine Ansicht des Trennkörpers aus Richtung des Pfeils VI - VI von Fig. 7; und
- Fig. 9: einen Längsschnitt der in einen Zentrifugenbecher mit Zentrifugeneinsatz eingesetzten Blutentnahmevorrichtung.

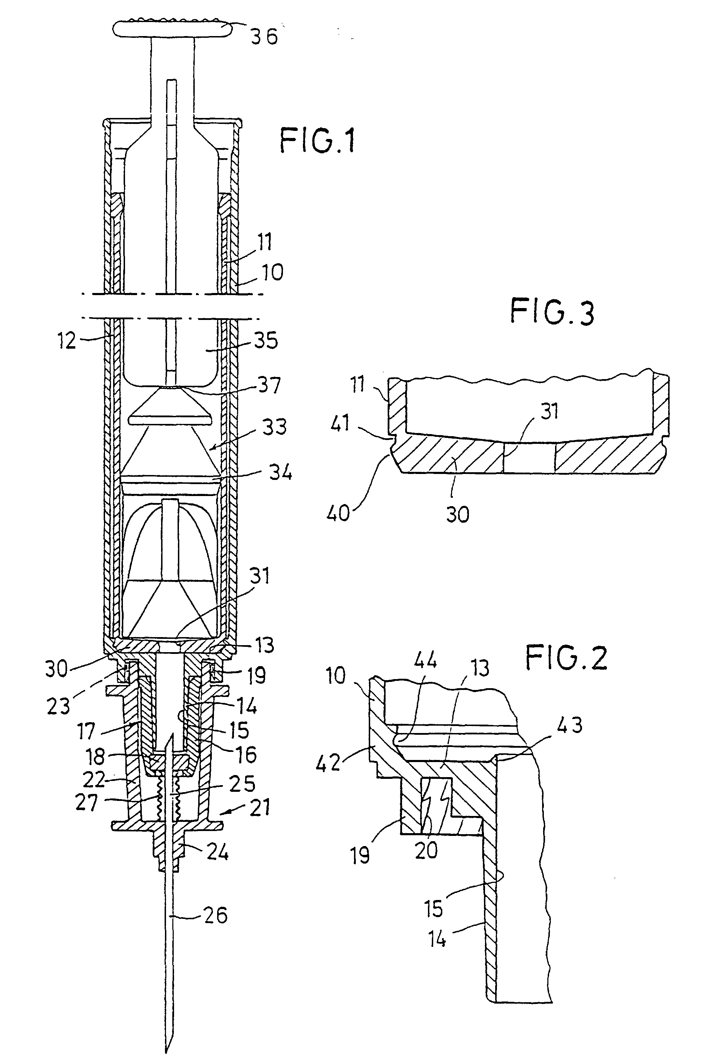

Die Blutentnahmevorrichtung weist gemäß Fig. 1 ein zylindrisches Außenröhrchen 10 aus einem relativ steifen, durchsichtigen Kunststoff auf, in dem ein ebenfalls durchsichtiges, zylindrisches Innenröhrchen 11 aus einem Material sitzt, das weicher ist als dasjenige des Außenröhrchens 10. Das Innenröhrchen 11 erstreckt sich nahezu über die gesamte Länge des Außenröhrchens 10. Zwischen Außenröhrchen 10 und Innenröhrchen 11 besteht ein Ringspalt 12, der eine radiale Aufweitung des Innenröhrchens ermöglicht.

Das Außenröhrchen 10 weist an seinem vorderen Ende eine Stirnwand 13 auf, die der Rohrwand des Röhrchens einstückig angeformt ist und von der ein zentrisch angeordneter Kanülenkonus 14 absteht. Durch den Kanülenkonus 14 erstreckt sich ein axialer Kanal 15. Auf dem Kanülenkonus 14 sitzt eine, von Hand abziehbare, aufgesteckte Kappe 16, die den Kanülenansatz 17 bildet. Diese Kappe 16 enthält eine den Kanal 15 verschließende elastomere Membran 18, die durchstochen werden kann.

Weiterhin befindet sich an der vorderen Stirnwand 13 des Außenröhrchens 10 ein umlaufender, nach vorne offener Ring 19, der an seiner Innenseite schräge Rippen 20 (Fig. 2) trägt. Der Ring 19 dient als Haltering für eine Mehrfachentnahmekanüle 21. Die Mehrfachentnahmekanüle 21 weist ein aus Kunststoff bestehendes, rohrförmiges Griffstück 22 auf, das axial verschiebbar und drehbar auf dem im wesentlichen zylindrischen Kanülenansatz 17 sitzt. Das Griffstück 22 ist mit Haltenocken 23 versehen, die zwischen die Rippen 20 des Ringes 19 greifen und durch Drehen des Griffstückes 22 mit den Rippen 20 verriegelt werden. Am vorderen Ende des Griffstückes 22 befindet sich eine Nabe 24, die eine beidseitig angeschärfte Kanüle 26 trägt, deren hinteres kürzeres Teil 25 in das Griffstück 22 hinein vorsteht. Das vordere Teil der Kanüle steht nach außen ab und dient zur Punktion der Vene. Das kürzere Kanülenteil 25 ist von einem Gummischlauch 27 umgeben. Wenn das Griffstück 22 auf den Kanülenansatz 17 aufgesteckt wird, durchdringt das hintere Kanülenteil den Schutzschlauch 27 und die Membran 18. Der Schutzschlauch 27 wird dabei vom vorderen Ende des Kanülenansatzes 17 zurückgehalten und ziehharmonikaartig zusammengedrückt. Nachdem das Griffstück 22 vollständig auf den Kanülenansatz 17 aufgeschoben ist, wird das Griffstück wie beschrieben im Ring 19 verriegelt und die Blutentnahme kann durchgeführt werden. Es besteht die Möglichkeit, statt der doppelendigen Mehrfachentnahmekanüle 21 eine normale Kanüle mit einem Aufnahmekonus am hinteren Ende auf die Blutentnahmevorrichtung aufzusetzen. Zu diesem Zweck wird die Verschlußkappe 16 vom Kanülenkonus 14 abgezogen und die normale Kanüle aufgesetzt.

Das vordere Ende des Innenröhrchens 11 weist eine Stirnwand 30 auf, die im wesentlichen ebenflächig ist und in der ein zentraler Durchgang 31 vorhanden ist, der mit dem Kanal 15 fluchtet.

In dem Innenröhrchen befindet sich der Trennkörper 33, der eine schmale ringförmige Kolbenfläche 34 aufweist, welche das Volumen des Innenröhrchens nach Art eines Kolbens in zwei getrennte Kammern unterteilt. An den übrigen Stellen ist der Durchmesser des Trennkörpers 33 kleiner als derjenige der Kolbenfläche 34. Das rückwärtige Ende des Trennkörpers 33 ist mit einer Kolbenstange 35 verbunden, die aus kreuzförmig angeordneten Rippen besteht und am rückwärtigen Ende eine Griffplatte 36 trägt. Die Kolbenstange 35 ragt aus den offenen Enden von Innenröhrchen und Außenröhrchen hinaus. Wenn sich der Trennkörper 33 in der vorderen Endstellung befindet, also gegen die Stirnwand 30 stößt, steht die Griffplatte 36 aus dem Außenröhrchen heraus vor. Durch Zurückziehen der Kolbenstange 35 und durch die abdichtende Wirkung der Kolbenfläche 34 erfolgt das Ansaugen von Blut in das Innenröhrchen hinein. Nach Beendigung dieser Aspiration wird die Kolbenstange 35 an einer Sollbruchstelle 37 von dem Trennkörper 33 abgebrochen. Der Trennkörper bildet sodann einen das Innenröhrchen verschließenden Stopfen.

Wie Fig. 3 zeigt, ist der Rand der Stirnwand 30 des Innenröhrchens 11 als wulstförmiges Rastprofil 40 ausgebildet, das am rückwärtigen Ende durch eine Einschnürung 41 in der Außenfläche der Umfangswand des Innenröhrchens 11 begrenzt ist. Dieses Rastprofil 40 wirkt mit einem Gegenprofil 44 zusammen, das an der Innenseite des Außenröhrchens 10 unmittelbar angrenzend an dessen Stirnwand 13 ausgebildet ist (Fig. 2). Das Gegenprofil 44 ist als ringförmige Mulde an der Innenseite eines verstärkten Bereichs 42 vorgesehen, der den Übergang von der Umfangswand des Außenröhrchens 10 zur Stirnwand 13 bildet. Der Außendurchmesser des Rastprofils 40 ist geringfügig größer als der Innendurchmesser des Gegenprofils 44. Das Rastprofil 40 und das Gegenprofil 44 bilden eine unter Vorspannung stehende Schnappverbindung, die einrastet, wenn das Innenröhrchen mit Kraft in das Außenröhrchen hineingedrückt wird. Wegen der Elastizität und Weichheit des Materials des Innenröhrchens 11 sind die ineinandergreifenden Profile 40 und 44 abdichtend. Andererseits hält das starre Gegenprofil 44 das Innenröhrchen 11 mit großer Kraft fest, so daß die Rastverbindung beim Aspirieren, d. h. beim Zurückziehen der Kolbenstange und des Trennkörpers, nicht gelöst wird.

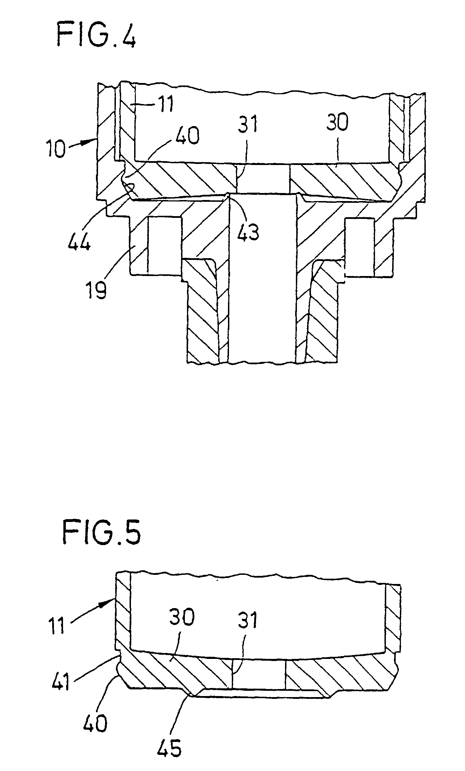

An der Stirnwand 13 des Außenröhrchens 10 ist eine ringförmig umlaufende spitze Erhebung 43 vorgesehen (Fig. 2), die den Kanal 15 eng umgibt und von der Stirnwand 13 axial in das Außenröhrchen hinein vorsteht. Wenn das Innenröhrchen fest in das Außenröhrchen hineingedrückt wird und die Schnappverbindung einrastet, liegt die Stirnwand 30 des Innenröhrchens unter Vorspannung auf der Erhebung 43 auf (Fig. 4). Während der Zentrifugation gräbt sich die Erhebung 43 in die Stirnwand 30 ein. Dadurch wird der Übergang von dem Kanal 15 zum Durchlaß 31 abgedichtet und es wird verhindert, daß Flüssigkeit in den Ringspalt 12 gelangen kann.

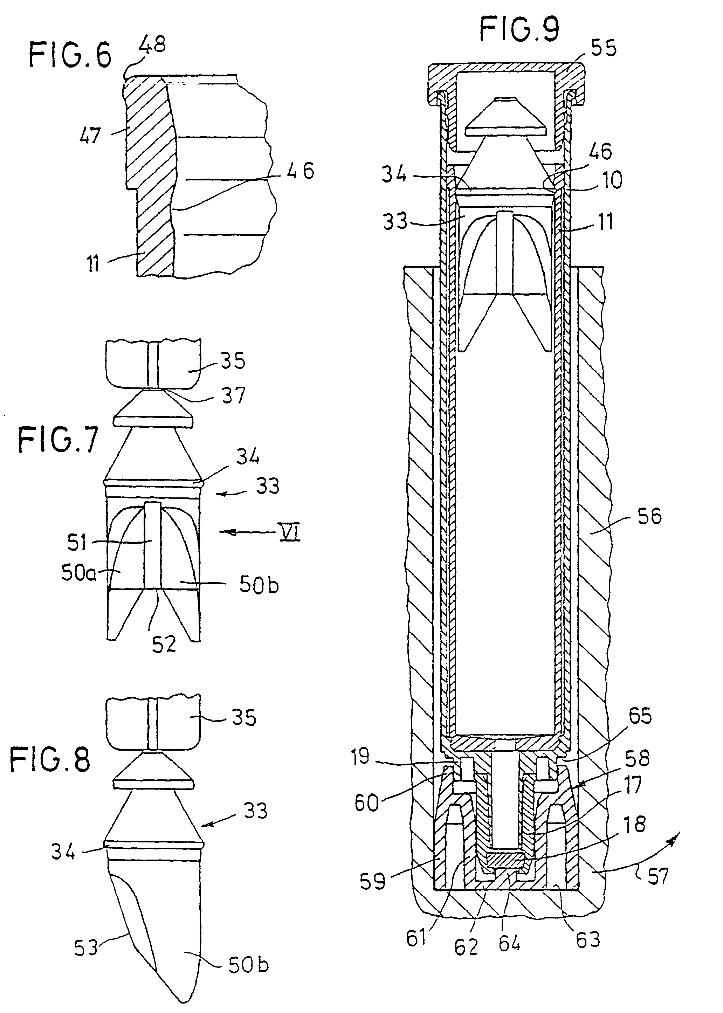

Bei dem in Fig. 5 dargestellten Ausführungsbeispiel ist eine ringförmige Erhebung 45 von vorzugsweise dreieckförmigem Querschnitt an der Außenseite der Stirnwand 30 des Innenröhrchens 11 vorgesehen. Diese Erhebung 45 wird während der Zentrifugation an der Stirnwand 13 des Außenröhrchens breitgedrückt und gewährleistet dadurch eine Abdichtung um den Durchlaß 31 herum.

Fig. 6 zeigt das rückwärtige Ende des Innenröhrchens 11. Das Innenröhrchen weist hier einen nach außen hin vorspringenden verdickten Bereich zur reibenden Abstützung an dem Außenrohr 10 auf. An der Innenseite des Innenröhrchens 11 ist eine Bremsnut 46 ausgebildet, die die Kolbenfläche 34 des Trennkörpers 33 in der rückwärtigen Endstellung rastend aufnimmt. In dieser Position kann die Kolbenstange 35 von dem Trennkörper 33 abgebrochen werden. Die Wirkung der Bremsnut 46 besteht darin, daß Herausziehen des Trennkörpers 33 zu vermeiden. Diese Wirkung wird durch den verdickten Bereich 47 verstärkt, der sich an dem Außenröhrchen 10 abstützt und ein Aufweiten des Innenröhrchens am rückwärtigen Ende verhindert. Dadurch wird ein versehentliches Herausziehen des steifen Trennkörpers 33 aus dem Innenröhrchen verhindert. Die Bremsnut 46 ist so ausgeführt, daß sie den Trennkörper 33 in der rückwärtigen Endstellung gegen den in dem Innenröhrchen gebildeten Unterdruck festhält. Somit kann die Blutentnahme auch nach der Vakuumentnahmetechnik erfolgen, bei der ein Unterdruck in dem Innenröhrchen 11 erzeugt wird, unmittelbar bevor die Blutentnahme erfolgt. Eine weitere Aufgabe des verdickten Bereichs 47 liegt in der rückwärtigen Abdichtung des Ringspaltes 12. Der Dichtring 48 verhindert, daß der durch die Zentrifugation gewonnene Serum/Plasmaüberstand bei der weiteren Probenverarbeitung in den Ringspalt 12 gelangt.

Der in den Figuren 7 und 8 dargestellte Trennkörper 33 ist als asymmetrischer Kippkörper gemäß EP 0 056 609 B1 ausgebildet, so daß er beim Zentrifugieren entsprechend der Schrägstellung des Zentrifugenbechers unterschiedliche Schrägstellungen in Bezug auf die Röhrchenachse einnehmen kann. Vor der als Ringwulst ausgebildeten Kolbenfläche 34 befinden sich zwei an ihren Enden spitz zulaufende Schenkel 50a, 50b, die durch einen Spalt 51 voneinander getrennt sind. Der Spalt 51 ist nur an einer Seite und nach vorne hin offen und an der Rückseite durch eine Umfangswand 52 verschlossen. Die Schenkel 50a und 50b haben zur Längsachse mindestens eine Abschrägung 53, die Schrägstellungen des Trennkörpers in dem aufgeweiteten Innenröhrchen ermöglicht. Der Trennkörper 33 besteht aus einem Material, dessen Dichte zwischen derjenigen der voneinander zu trennenden Blutphasen liegt.

Das Außenröhrchen 10 kann durch Aufsetzen einer Verschlußkappe 55 (Fig. 9) verschlossen werden. Die Verschlußkappe sitzt nur in dem Außenröhrchen und dichtet das rückwärtige Ende des Außenröhrchens ab. Da dieses rückwärtige Ende über das Innenröhrchen hinaus vorsteht, kommt die Verschlußkappe mit dem Innenröhrchen nicht in Berührung. Nach dem Aspirieren des Blutes in das Innenröhrchen sitzt der Trennkörper 33 mit seiner Kolbenfläche 34 in der Bremsnut 46 fest, um das Innenröhrchen am rückwärtigen Ende zu verschließen. Die Verschlußkappe 55 bildet eine zusätzliche Abdeckung. Das Zentrifugieren mit aufgesetzter Verschlußkappe ist bei bestimmten Zentrifugenausführungen zur Vermeidung des Aerosoleffektes sinnvoll.

In Fig. 9 ist ein Zentrifugenbecher 56 dargestellt, der mit radialem Abstand von der Drehachse eines Zentrifugenrotors hängend angeordnet ist. Bei Drehung des Rotors wird der Zentrifugenbecher mit seinem unteren Ende in Richtung des Pfeils 57 hochgeschwenkt, so daß er schließlich eine annähernd horizontale Lage einnimmt. Auf dem Boden des Zentrifugenbechers 56 ist ein Zentrifugeneinsatz 58 angeordnet, der aus starrem Kunststoff besteht. Der Zentrifugeneinsatz 58 hat eine Umfangswand 59, mit der er im Zentrifugenbecher 56 zentriert wird. Von der Umfangswand 59 ragt ein Ringkragen 60 auf, der den Ring 19 des Außenröhrchens 10 zentrierend umgibt. Die Umfangswand 59 umschließt eine ringförmige Zentrierwand 61, die den Kanülenansatz 17 zentrierend aufnimmt. Die Zentrierwand 61 ist mit einer Bodenwand 62 verbunden, welche sich auf dem Boden 63 des Zentrifugenbechers 56 abstützt. Von dieser Bodenwand 62 steht ein zentrischer Zapfen 64 ab, der in das vordere Ende des Kanülenansatzes 17 eindringt und an der Membran 18 abstützend anliegt. Der Zentrifugeneinsatz 58 sitzt beweglich in dem Zentrifugenbecher und kann bei Bedarf (z. B. zur Reinigung) herausgenommen werden.

Während der Zentrifugation drückt sich der aus Außenröhrchen und Innenröhrchen bestehende Behälter infolge der Zentrifugalkraft gegen den Zentrifugeneinsatz 58. Das Außenröhrchen 10 liegt mit einer den Ring 19 umgebenden Ringschulter 65 auf der Stirnfläche des Zentrierringes 60 auf. Gleichzeitig drückt sich der Zapfen 64 in die Gummimembran 18. Dabei wird die Gummimembran 18 gegen den Druck der Flüssigkeitssäule zusammengepreßt und dichtet den Behälter ab, so daß keine Flüssigkeit in den Zentrifugenbecher 56 hinein austreten kann.

Zusätzlich zu der äußeren Abdichtung des Behälters erfolgt die innere Abdichtung durch die ringförmige Erhebung 43 (Fig. 2) bzw. 45 (Fig. 5) sowie durch die ineinandergreifenden Rastprofile 40 und 44 (Fign. 2 und 3). Dadurch wird verhindert, daß Flüssigkeit in den Ringspalt 12 eintritt und die Aufweitung des weichen Innenröhrchens 11 verhindern würde. Infolge der Aufweitung des Innenröhrchens 11 beim Zentrifugieren dichtet die Kolbenfläche 34 des Trennkörpers 33 nicht mehr ab. Der Trennkörper nimmt eine Position zwischen den beiden zu trennenden Blutphasen ein. Nach Beendigung der Zentrifugation zieht sich das Innenröhrchen 11 wieder zusammen und legt sich dichtend um die Kolbenfläche 34 des Trennkörpers 33.

Die Verschlußkappe 55 kann so ausgeführt sein, daß sie von einer Probennadel durchstochen werden kann. Dies ermöglicht eine Probenentnahme im geschlossenen System.

Die Blutentnahmevorrichtung kann alternativ zu dem vorstehend beschriebenen Ausführungsbeispiel ein Vakuum enthalten, um das Blut einzusaugen. In diesem Fall ist im Innenröhrchen nur der Trennkörper 33 vorhanden und die Kolbenstange 35 ist entbehrlich.

## Patentansprüche

1. Blutentnahmevorrichtung mit einem Außenröhrchen (10), das eine Stirnwand (13) mit Kanülenansatz (17) aufweist, und einem Zentrifugeneinsatz (58), der eine Aufnahme für den Kanülenansatz (17) aufweist, wobei in der Aufnahme ein Zapfen (64) angeordnet ist,
**dadurch gekennzeichnet,**
**dass** der Kanülenansatz (17) eine durchstechbare Membran (18) enthält und der Zapfen (64) zum Abstützen der Membran (18) ausgebildet ist.

2. Blutentnahmevorrichtung nach Anspruch 1, **dadurch gekennzeichnet, dass** der Zentrifugeneinsatz (58) eine Umfangswand (59) aufweist, von der ein Ringkragen (60) aufragt, der einen Ring (19) des Außenröhrchens (10) zentrierend umgibt.

## Claims

1. Blood collection device having an outer tube (10) comprising a front wall (13) with a cannula hub (17), and a centrifuge insert (58) comprising a receiving means for the cannula hub (17), wherein a journal (64) is arranged in the receiving means,
**characterized in that**
the cannula hub (17) contains a membrane (18) adapted to be perforated, and the journal (64) is configured to support the membrane (18).

2. Blood collection device according to claim 1, **characterized in that** the centrifuge insert (58) comprises a circumferential wall (59) from which an annular collar (60) extends which surrounds a ring (19) of the outer tube (10) for the centering thereof.

## Revendications

1. Dispositif de prise de sang comprenant un tube externe (10), lequel comporte une paroi frontale (13) avec un embout pour canule (17), et un insert de centrifugation (58), lequel présente un évidement pour l'embout pour canule (17), un pion (64) étant agencé dans ledit évidement, **caractérisé en ce que** l'embout pour canule (17) comprend une membrane (18) pouvant être perforée et que le pion (64) est conçu pour recevoir la membrane (18) en butée.

2. Dispositif de prise de sang selon la revendication 1, **caractérisé en ce que** l'insert de centrifugation (58) comporte une paroi périphérique (59), dont une collerette annulaire (60) fait saillie, laquelle entoure un anneau (19) du tube externe (10) de manière à le centrer.
